# EUROPEAN PATENT SPECIFICATION

(11) **EP 2 491 977 B1**
(45) Date of publication and mention of the grant of the patent: **08.01.2014**
(21) Application number: 11156295.5
(22) Date of filing: 28.02.2011
(51) Int. Cl.: A61N 1/362, A61N 1/365

(54) **System for adapting pacing settings of a cardiac stimulator**
System zur Anpassung von Stimulationseinstellungen eines Herzschrittmachers
Système d'adaptation des paramètres de stimulation d'un stimulateur cardiaque

(43) Date of publication of application: 29.08.2012
(73) Proprietor: St. Jude Medical AB, 175 84 Järfälla (SE)
(72) Inventor: Karlsson, Andreas, SE-169 67, Solna (SE); Blomqvist, Andreas, SE-187 76, Täby (SE); Järverud, Karin, SE-169 71, Solna (SE)
(74) Representative: Sjölander, Henrik

(56) References cited:
- EP-A2- 0 503 839
- EP-A2- 1 486 232
- WO-A1-2010/024739
- US-A1- 2006 100 668
- US-A1- 2008 082 001
- US-B1- 7 139 609
- US-B1- 7 333 854
- DATABASE MEDLINE [Online] US NATIONAL LIBRARY OF MEDICINE (NLM), BETHESDA, MD, US; December 1990 (1990-12), REES M ET AL: "Effect of rate-adapting atrioventricular delay on stroke volume and cardiac output during atrial synchronous pacing.", XP002646464, Database accession no. NLM2272000

## Description

### Technical field

The present invention generally relates to implantable medical devices, such as cardiac stimulators including e.g. pacemakers, and, in particular, to systems for predicting patient responses to physical exertion, monitoring the patient response over time to evaluate disease progression and adapting pacing therapies of cardiac stimulators based on the predicted patient response.

### Background of the invention

Heart failure (HF) is a growing medical challenge and worldwide epidemic. Heart failure can have many different reasons and pathophysiologies. The complexity and difficulties associated with heart failure has captured the attention of the cardiology community substantially for the last decade. In clinical practice today, many patients can be managed effectively through pharmacological therapy such as beta-blockers, ACE inhibitors, and diuretics. However, since the introduction of CRT devices they have served as a compliment to drug therapy for heart failure patients. In screening for HF patients the echocardiography measure ejection-fraction (EF) has long since been used to evaluate the systolic function of the patient. If a patient shows up with the traditional heart failure symptoms such as fatigue, shortness of breath, excessive fluid retention etc and has an EF below 30 or 35 %, they are in most cases considered to suffer from heart failure.

HF patients usually have rather long periods during which they are at a more or less stable level of their disease, during which no apparent change of their condition is present. However, sooner or later some kind of precipitating event occurs that will trigger a worsening of the disease and they can exacerbate very rapidly and may have to be hospitalized for treatment, quite often with pulmonary edema. These precipitating events may be an ischemic event, another disease, non-compliance to drug therapy, etc. Another triggering event may be physical exertion. The tolerance to physical exertion varies greatly between heart failure patients depending on, inter alia, disease status and on the patient's etiology. Although many heart failure patients are eligible for a CRT device it is not obvious that all of them would benefit from the same therapy. Certainly this is where the electrophysiologist's or heart failure specialist's expertise comes into play. Currently, medical history and physical examination are the most important tools that a physician uses to determine and mark the progress of a heart failure patient.

It would be useful if methods and devices were available that help a physician or other medical personnel or to objectively evaluate and tailor therapy for the patient as well as evaluate and monitor heart failure progression (or regression). Further, it would also be useful if methods and devices were available that automatically could adapt the therapy to, for example, cope with changes over time in the level of disease to thereby tailor the therapy for a heart failure patient to allow them to live a more normal life in comparison with a "one size fits all" therapy offered today be many prior art device.

In US patent 6,980,851 methods and devices for continuously monitoring a heart failure status of a patient are shown. By combining parameters related to heart failure status, for example, parameters derived from sensing signals such as QRS duration, heart rate variability, PR interval, frequency of atrial fibrillation, or frequency of ectopic beats with other parameters such as body temperature during exercise, and changes in activity levels, a clinical state vector that represents an estimate of the patient's current heart failure status can be created. A difference between a presently computed state vector and a previously computed state vector represents a clinical trajectory that indicates if and to what degree a patient's heart failure status has changed. Hence, this approach may provide an indication over disease changes that may assist a physician when evaluating the therapy.

EP 0 503 839 relates to regulating blood flow within the cardiovascular system in a closed-loop control system using ultrasound measurement techniques to determine a hemodynamic status of the patient and to derive a control parameter for modulating the hemodynamics of the system using electrical or pharmaceutical therapy.

EP 1 486 232 discloses a device for decreasing workload, maintaining or increasing cardiac output and/or achieving a desirable autonomic balance by stimulating one or more parasympathetic nerves.

US 2006/0100668 discloses a control unit that drives an electrode device to apply a stimulating current to an autonomic nerve of a patient. The stimulating current is capable of inducing action potentials in a therapeutic direction in a first set and a second set of nerve fibers of the nerve. The electrode device also applies an inhibiting current that is capable of inhibiting the induced action potentials travelling in the therapeutic direction in the second set of nerve fibers.

US 2008/0082001 discloses an implantable medical device with a controller circuit that senses a change in a physiological signal as a result of a change in posture and generates a response as a function of that change.

US 7,333,854 discloses measuring cardiac output after detection of a change in position and based at least in part on measuring cardiac output. It is then decided whether to increase a cardiac pacing rate.

WO 2010/024739 discloses an implantable heart monitoring device configured to monitor the status of the heart. The device derives information correlated to stroke volume at a detected heart rate. Information that indicates the stroke volume as a function of the heart rate is derived.

US 7,139,609 estimates patient cardiac function in terms of stroke volume, cardiac output or maximum rate of change of aortic pressure with time based on internal measurements of heart sounds. Control parameters of a medical device are then automatically adjusted so as to optimize overall cardiac function or to provide for ventricular resynchronization therapy.

Rees M. et al., Effect of rate-adapting atrioventricular delay on stroke volume and cardiac output during atrial synchronous pacing, The Canadian Journal of Cardiology, 1990, 6(10):445-452 suggests that patients with atrial synchronous pacemakers have a variable stroke volume response to exercise. It appeared that in the patients the ability to increase heart rate was the key factor for raising cardiac output during exercise.

However, there is still a need within the art for methods and devices that are capable of automatically and continuously providing patient unique therapy that can assist the patient in living a more normal life than with prior art devices.

### Summary of the invention

An object of the present invention is to provide a system that is capable of automatically and continuously providing patient unique therapy based on predicted patient response to physical exertion.

Another object of the present invention is to provide a system for adapting pacing settings of a cardiac stimulator based on predicted patient response to physical exertion. In particular, the pacing settings are adapted based on a cardiac status indicator indicating a primarily heart rate response or a primarily stroke volume response to increased workload.

These and other objects are achieved with a system as defined by the independent claims. Preferred embodiments and variation of the present invention are defmed by the dependent claims.

Disclosed herein, but not forming part of the invention, is a method for adapting a pacing therapy of a cardiac stimulator implanted in a patient. The method comprises determining a cardiac response of the heart of the patient to changed activity using hemodynamical signal measured at each of at least two different levels of physical activity, wherein at least one level of physical activity corresponds to an increased activity level compared to an activity level at rest. Further, a current cardiac status indicator for the patient is created using the cardiac response, wherein the cardiac status indicator indicates a response of the patient to an increased physical activity as a primarily heart rate response or as a primarily a stroke volume response. The pacing parameters of the cardiac stimulator can thereafter be adapted depending on the current cardiac status indicator, wherein the adapted pacing parameters include a first pacing setting if the current cardiac status indicator indicates a primarily heart rate response or a second pacing setting if the current cardiac status indicator indicates a primarily stroke volume response. When the adaptation process is finished, the new pacing parameters are used by the cardiac stimulator for pacing the heart of the patient until the next adaptation process has been executed, possible resulting in an updated set of pacing parameters, which then replaces the current pacing parameters.

According to an aspect of the present invention, there is provided a system for adapting a pacing therapy of a cardiac stimulator implanted in a patient. The system comprises an activity detector configured to detect at least one activity parameter reflecting physical activity of the patient and a hemodynamical measuring module configured to measure hemodynamical signals at each of at least two different levels of physical activity the signals reflecting a cardiac response of the heart of the patient to changed activity, wherein at least one level of physical activity corresponds to an increased activity level compared to an activity level at rest. Further, a cardiac status indicator module is configured to create a current cardiac status indicator for the patient using the cardiac response, wherein the cardiac status indicator indicates a response of the patient to an increased physical activity as a primarily heart rate response or as a primarily a stroke volume response. A pacing parameter determining module is configured to determine a pacing parameter setting based on the current cardiac status indicator. The adapted pacing parameters include a first pacing setting if the current cardiac status indicator indicates a primarily heart rate response or a second pacing setting if the current cardiac status indicator indicates a primarily stroke volume response and to instruct a timing circuitry and/or a pacing module of the cardiac stimulator to adapt pacing parameters depending on the current cardiac status indicator. The adapted pacing parameters include a first pacing setting if the current cardiac status indicator indicates a primarily heart rate response or a second pacing setting if the current cardiac status indicator indicates a primarily stroke volume response.

The present invention is based on findings with regard to cardiac response, and especially with regard to cardiac output, of patients, and particularly heart failure patients, to increased metabolic demands, for example, in connection with physical effort. The inventors have inter alia discovered two patient groups displaying significant differences in heart behavior (or cardiac response) at different activity levels. Moreover, the inventors have also found that each of these groups is characterized by clinically distinct changes in hemodynamical parameters. In particular, it was found that within a first group of patients, the increase in cardiac output resulting from the physical effort mainly was achieved by an increased heart rate. That is, within this first patient group, the response to the physical effort was an increased heart rate and a more or less stable (or un-changed) stroke volume or only a very low increase of the stroke volume. Further, in the second group, the increased cardiac output was mainly achieved by an increased stroke volume while the heart rate was substantially un-changed, or showed a very low increase. Accordingly, the inventors have found two hemodynamical parameters that provide valuable information regarding the heart behavior of heart failure patients at different activity levels. Further, the inventors have found two groups of patients in which the heart behaves in distinctly different ways at an increased activity level. The responses identified within these patient groups are in sharp contrast to the expected response which is an increased heart rate and stroke volume.

These findings can, for example, be used to predict patient response, or heart response, to physical exertion or exercise. For a patient classified as belonging to the first group, a predicted cardiac response to cope with the increased cardiac output resulting from exercise is a heart rate increase and a more or less un-changed stroke volume. Similarly, for a patient classified as belonging to the second group, a predicted cardiac response to exercise is instead an increased stroke volume and a more or less stable heart rate. Furthermore, patient response to physical exertion or exercise can be monitored over time in order to identify, for example, disease progression. Over time a specific patient can move within a group and even from one group to the other. Of course, many patients have a combination of the two extreme responses but where one of the responses, i.e. primarily heart rate response or primarily stroke volume response, is more pronounced.

These findings can also be used to tailor-make or customize exercise-modes for heart failure patients to obtain a better adaptation of the therapy to the behavior of their heart.

The inventors have further discovered that the patients in the first group all were diagnosed with ischemic cardiomyopathy and all patients in the second group were diagnosed with dilated cardiomyopathy. Thus, the present findings can be used to classify patient etiology and to monitor a progression of the disease in question.

The present invention can thus be used to inform the physician and/or the patient in which direction the patient characteristics is moving and provide valuable information on which aspects of the patient characteristics that is more important to monitor for a particular patient.

Furthermore, the present invention provides an on-demand tool for the physician enabling a monitoring whether if a certain pacemaker setting, drug or other means of therapy can influence the parameter to move in a desired way and it also possible to check the status of the patient in a qualitative way.

The customized exercise mode or mode for increased physical activity can be tailored for the two identified groups. If a person is unable to respond to an increased metabolical need by increasing stroke volume, the patient is classified as likely to respond by an exaggerated response in the heart rate and an exercise mode is thus adapted to handle this. If instead a patient is capable of increasing stroke volume but show signs of chronotropic incompetence, it may be appropriate to instead increase the slope of the rate response. The slope is the sensitivity of the change of stimulation frequency as a response to a certain change of the physical activity. Patients with a primarily heart rate response to an increased workload may have settings with a lower maximum track rate, as these patients may have a tendency to rush the heart and thereby worsen their (often) ischemic condition. The maximum track rate is the highest ventricular stimulation rate that will follow detected intrinsic atrial rate. The settings of the exercise mode can be set automatically by the cardiac stimulator in an adaptation procedure or session or may be decided exactly by the managing physician.

It also conceivable to implement an exercise alert informing the patient, for example, by means of a vibrator device, which helps or assists the patient to even dare to try to exercise or increased the level of physical extortion. It also possible to apply optimized Bi-ventricular pacing for ischemia as the patient is classified in this group.

For patients with a primarily stroke volume response, a possible setting is a setting that promotes an increased heart rate in connection with activity by increasing the slope of the rate response setting such that a higher rate is employed during physical exertion. Patients in this group often has a dilated cardiomyopathy and are likely to be very sensitive to myocardial wall stress, a condition caused by overload of blood volume or pressure. By using blood volume controlled pacing as described in US patents 5,417,715 and 6,078,835 by the same applicant, this can be managed.

According to an embodiment of the present invention, the cardiac response is determined by the cardiac stimulator based on recorded hemodynamical signals at each of at least two different levels of physical activity. The hemodynamical signals are according to one embodiment, cardiac impedance signals measured by different impedance vectors. One vector configuration includes two impedance vectors. In the first impedance vector, current is injected between a right atrium ring electrode and a right ventricle ring electrode and the resulting voltage is measured between a right atrium tip electrode and a right ventricle tip electrode. In the second vector, the current is injected between a right ventricle ring electrode and a right ventricle tip electrode and the resulting voltage is measured over the same electrodes. The cardiac impedance signals obtained by these vectors divulge information about the local (first vector) and global (second vector) global cardiac contraction characteristics. Based on these cardiac impedance signals different impedance parameters can be extracted, for example, slope of the waveform in a certain portion of the signal, peak-to-peak value of the waveform or area of a certain portion of the waveform.

Alternative hemodynamical signals include blood pressure, for example, measured by means of a pressure sensor attached to septum, which enables measurements of blood pressure in left ventricle. A suitable pressure sensor is, for example described, in the co-pending application PCT/EP2010/058624 by the same applicant, US RE 39,863, US 6,248,083, or US RE 35,648. Other types of hemodynamical sensors may, alternatively or as a complement, be used including accelerometers for measuring pressure changes in the left ventricle, flow probes, load indicators for measuring geometrical changes in the cardiac tissue e.g. in septum, heart sound sensors, or photoplethysmographic sensors.

Based on the extracted hemodynamical parameters, the cardiac response indicator can be created or calculated. In a preferred embodiment, the cardiac response indicator is calculated by linearly combining a number of impedance based parameters in a multivariate model.

According to embodiments of the present invention, the cardiac response is determined by observing a cardiac response of the heart at each of the at least two different levels of physical activity including observing heart rate response and/or stroke volume response to the changed activity using external equipment. The physician can thus, based on the observations using the external equipment, determine the cardiac response. The observations can be performed by means of external equipment capable of communicating with the cardiac stimulator and/or by means of external equipment that can perform hemodynamical measurements in the heart or at other location of the body of the patients to obtain hemodynamical information reflecting the functioning of the heart. For example, any one of the following types of equipment can be used:
- non-invasive echocardiography/ultrasound equipment: An example of a parameter is aortic velocity time integral (correlates with stroke volume (SV))
- Photoplethysmograpic technique. Using this technique, for example, stroke volume and LV dP/dtₘₐₓ can be obtained.
- Non-invasive equipment for measurement using the fingers of the patient.

Using this technique, for example, stroke volume and LV dP/dtₘₐₓ can be obtained.
- Non-invasive magnetic resonance imaging (MRI), for example ejection-fraction or stroke volume can be derived.
- Swan-Ganz catheter: This is a standard method for monitoring cardiac output. Based on the measured cardiac output, stroke volume can be determined by dividing the cardiac output with heart rate.
- Catheter based equipment for measuring pulmonary capillary wedge pressure (left atrial pressure surrogate right atrial pressure). This catheter can also be used to measure cardiac output.
- Millar catheter or Radi pressure wire for measuring left ventricular pressure:
- Equipment for impedance cardiography: For example, stroke volume (SV) or cardiac output (CO) can be obtained.

According to embodiments of the present invention, an adaptation of therapy is triggered by detection of at least one event related to the activity parameter, for example, when a predetermined limit is exceeded, by receipt of an instruction to adapt the pacing parameters, for example, sent manually by a physician via a programmer unit capable of communicating with the cardiac stimulator via telemetry, or at regular intervals of time, for example, once a week.

It is possible to incorporate the use of other hemodynamic sensors, such as e.g. a pressure sensor located in left atrium for measuring left atrium pressure, in the exercise application. For example, patients that are prone to display large increases in heart rate are also likely to have an impaired diastolic filling, due to ischemic heart and fibrotic tissue in the myocardium resulting in a stiffer heart. For these patients filling pressures are imperative for the little diastolic function they have left. With a rapidly increasing heart rate the result may be that filling pressures decline resulting in an abnormally small end diastolic volume, which may yield an extremely high ejection fraction but virtually zero stroke volume. In this case, a left atrium pressure sensor could indicate when filling pressures are declining too rapidly and an alert can be initiated (and thus informing the patient and/or the clinic), but up until that point the increase in heart rate would be tolerated.

In embodiments of the present invention, the detection of at least one activity parameter comprises detecting a cardiac output and/or a change of a cardiac output of the patient, and/or an activity level and/or a change of the activity level of the patient, and/or a heart rate and/or a change of the heart rate of the patient.

According to embodiment of the present invention, the cardiac status indicator is associated with a value on a scale ranging from a first end value corresponding to a cardiac status indicator indicating a response of the patient to an increased physical activity as a primarily heart rate response to a second end value corresponding to a cardiac status indicator indicating a response of the patient to an increased physical activity as a primarily stroke volume response. In this case, the pacing parameters can be adapted depending on the value on the scale of the current cardiac status indicator, wherein a maximum track rate is lower the closer a value of the cardiac status indicator is to the first end value, and wherein a slope of a rate response setting is steeper the closer a value of the cardiac status indicator is to the second end value.

Patients lacking an ability to produce a cardiac rhythm, for example, due to block in the heart's electrical conduction system cannot respond to an increased metabolic need by increasing the heart rate has probably corresponding need as the patients having chronotropic incompetence and therefore the rate responsive function is important for these patients. For these patients, there is no reason to monitor whether the ability to respond to physical exertion by means of a heart rate increase since they cannot develop or improve this ability. However, it may be interesting to monitor their ability to increase the stroke volume in response to increased workload.

Further objects and advantages of the present invention will be discussed below by means of exemplifying embodiments.

These and other features, aspects and advantages of the invention will be more fully understood when considered with respect to the following detailed description, appended claims and accompanying drawings.

### Brief description of the drawings

Exemplifying embodiments of the invention are illustrated by way of example and not by way of limitation in the figures of the accompanying drawings in which like references indicate similar elements. It should be noted that references to "an" or "one" embodiment in this discussion are not necessarily to the same embodiment, and such references mean at least one.
Fig. 1 is a schematical diagram illustrating a system according to the present invention can be implemented.
Fig. 2 is a simplified diagram of one embodiment of an implantable stimulation device in electrical communication with several leads implanted in a patient's heart for measuring hemodynamical signals delivering multi-chamber stimulation.
Fig. 3 is a simplified functional block diagram of one embodiment of a cardiac stimulator in accordance with the present invention.
Fig. 4 is a diagram illustrating how the cardiac status indicator can separate patient groups.
Fig. 5 is a flow chart illustrating general steps of a method for adapting pacing settings of a cardiac stimulator according to the present invention.

### Description of exemplifying embodiments

The following is a description of exemplifying embodiments in accordance with the present invention. This description is not to be taken in limiting sense, but is made merely for the purposes of describing the general principles of the invention. It is to be understood that other embodiments may be utilized and structural and logical changes may be made without departing from the scope of the present invention.

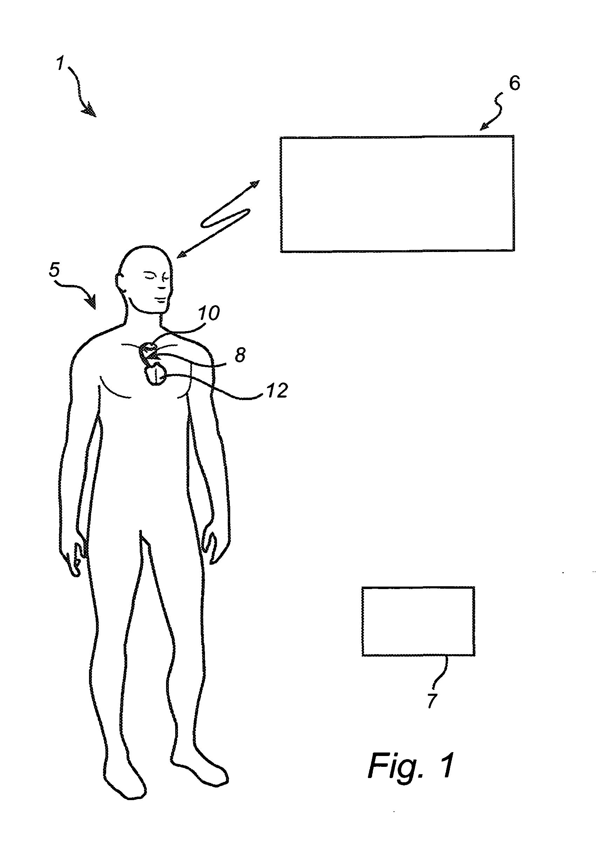

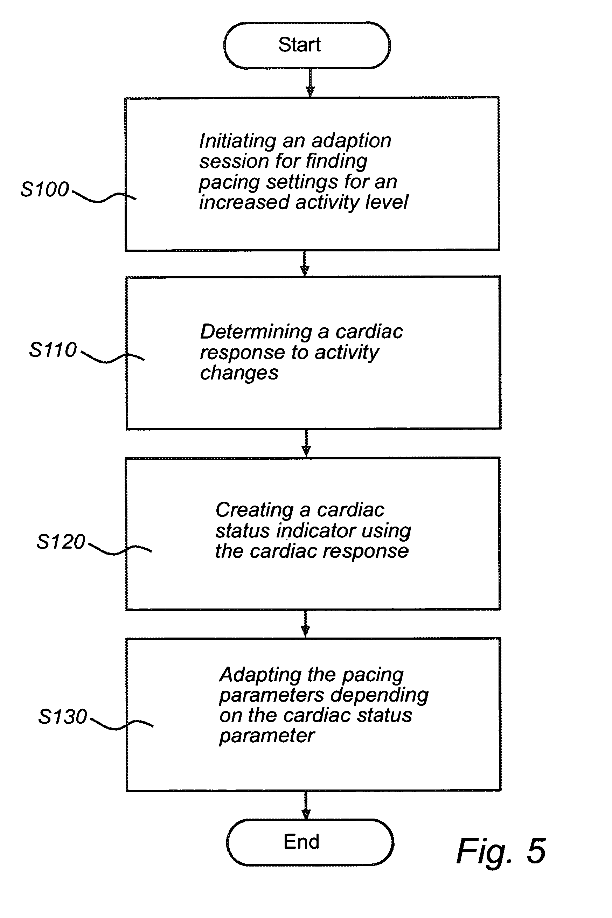

Referring to Fig. 1, an embodiment of a system environment according to the present invention will be described. A method of using the system of the present invention is briefly described with reference to Fig. 1 but will be described in more detail with reference to Fig. 5.

With reference first to Fig. 1, an embodiment of a system 1 according to the present invention for adapting a pacing therapy of cardiac stimulator 10 implanted in a patient 5 will be discussed. The present invention is based on the findings that in particular two patient groups displaying significant differences in heart behaviour (or cardiac response) at different activity levels. A first group of patients respond to an increase in physical exertion by primarily an increased heart rate. That is, within this first patient group, the response to the physical effort was an increased heart rate and a more or less stable (or un-changed) stroke volume or only a very low increase of the stroke volume. In a second group, the increased cardiac output is mainly achieved by an increased stroke volume while the heart rate is substantially un-changed. Thus, this can be used to predict patient response to physical exertion, to monitor disease progression or to tailor exercise modes for heart failure patients. An adaptation process to implement an exercise mode can be initiated or triggered automatically at detection of at least one event related to an activity parameter detected at the patient, by receipt of an instruction to adapt the pacing parameters received from an external unit 6 such as a programmer unit, or at regular intervals of time. For example, the physician may manually via the programmer unit 6 initiate an adaptation session at a follow-up visit by a patient 5 carrying an implanted cardiac stimulator 10 at a clinic. Preferably, such a manually initiated adaptation session is performed under supervision of the physician.

According to the present invention, a cardiac status indicator indicating a response of the patient to an increased physical activity as a primarily heart rate response or as a primarily a stroke volume response is created. The cardiac status indicator, as will be explained in more detail below, is created based on recorded hemodynamical parameters at two different levels of physical activity or based on observations of a cardiac response of the heart 5 at different levels of physical activity by the physician. For example, the physician may observe a heart rate response and/or stroke volume response to changed activity via external monitoring equipment 7 configured to communicate electrically with, for example, the heart 12 and/or other organs of the patient 5, e.g. a device for measuring a cardiac impedance of the heart 12. The external monitoring equipment 7 may alternatively or also be to communicate with the cardiac stimulator 10 to gather hemodynamical data, e.g. cardiac impedance data, measured by a hemodynamical measuring module 47 of the cardiac stimulator.

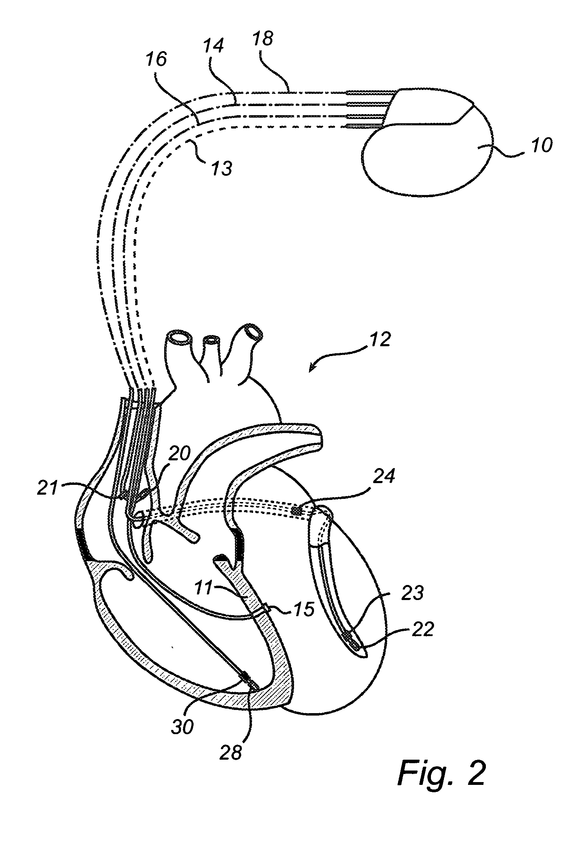

The cardiac stimulator 10 is connectable to one or more medical leads 8. The implantable cardiac stimulator 10 can thereby be set in electrical communication with the patient's heart 12 by way of the leads 8, for example, suitable for delivering multi-chamber stimulation therapy. In Fig. 2 an example cardiac stimulator is shown in electrical communication with the patient's heart 12 by way of three leads 14, 16, and 18 suitable for delivering multi-chamber stimulation therapy.

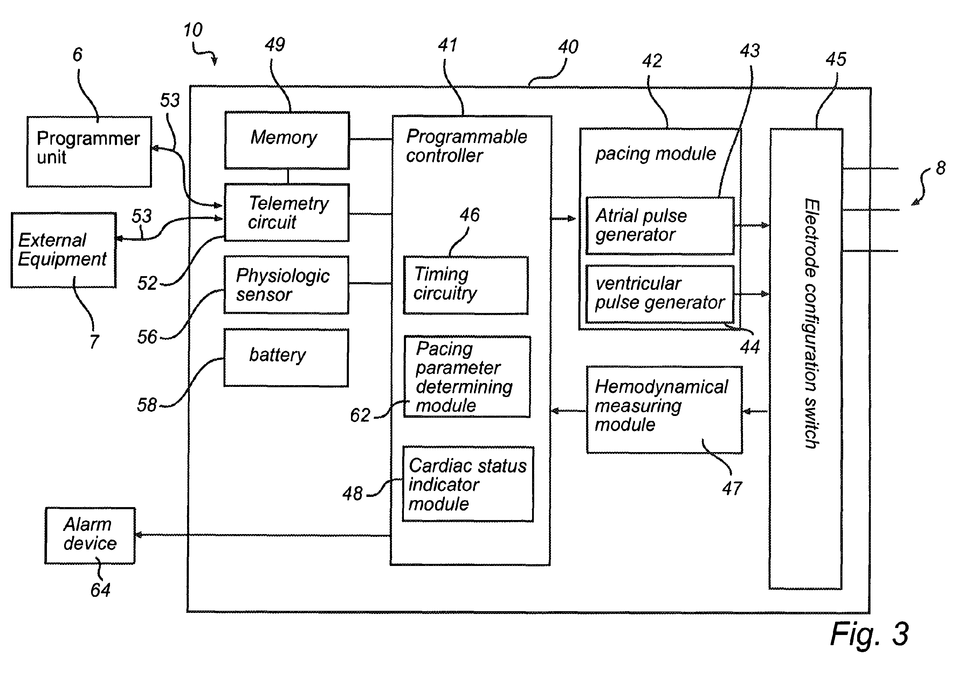

In order to gather hemodynamical data, the cardiac stimulator 10 includes a hemodynamical measuring module 47 (see Fig. 3), which may be connected to a hemodynamical sensor external to the cardiac stimulator 10. An example of such an external hemodynamical sensor is a pressure sensor 15 attached to septum 11 which enables measurements of blood pressure in left ventricle. A suitable pressure sensor is, for example described, in the co-pending application PCT/EP2010/058624 by the same applicant, US RE 39,863, US 6,248,083, or US RE 35,648. An example of a hemodynamical measuring module 47 of the cardiac stimulator 10 is an impedance measuring module configured to measure cardiac impedance via electrodes of the medical leads 14, 16, and 18.

However, other types of hemodynamical sensors may, alternatively or as a complement, be used including accelerometers for measuring pressure changes in the left ventricle, flow probes, load indicators for measuring geometrical changes in the cardiac tissue e.g. in septum, heart sound sensors, or photoplethysmographic sensors.

To sense atrial signals and to provide right atrial chamber stimulation therapy, the stimulator 10 is coupled to an implantable right atrial lead 14 having, for example, an atrial tip electrode 20, which typically is implanted in the patient's right atrial appendage or septum. Fig. 1 shows the right atrial lead 14 as also having an atrial ring electrode 21.

To sense left atrial and ventricular cardiac signals and to provide left chamber pacing therapy the stimulator is coupled to a coronary sinus lead 16 designed for placement in the coronary sinus region via the coronary sinus for positioning a distal electrode adjacent to the left ventricle and/or additional electrode(-s) adjacent to the left atrium. As used herein, the phrase "coronary sinus region" refers to the vasculature of the left ventricle, including any portion of the coronary sinus, great cardiac vein, left marginal vein, left posterior ventricular vein, middle cardiac vein, and/or small cardiac vein or any other cardiac vein accessible via the coronary sinus.

The lead 16 is designed to receive atrial and ventricular cardiac signals and to deliver left ventricular pacing therapy using, for example, a left ventricular tip electrode 22, a left ventricular ring electrode 23, and left atrial pacing therapy using, for example, a left atrial ring electrode 24.

The cardiac stimulator 10 is also in electrical communication with the heart 12 by way of an implantable right ventricular lead 18 having, in this embodiment, a right ventricular tip electrode 28 and a right ventricular ring electrode 30. Typically, the right ventricular lead 18 is transvenously inserted into the heart 12 to place the right ventricular tip electrode 28 in the right ventricular apex. The right ventricular lead 18 is capable of sensing or receiving cardiac signals, and delivering stimulation in the form of pacing therapy.

The impedance measuring module may measure cardiac impedance using two impedance vectors. A first impedance vector is in a preferred embodiment:
- Current injection: RA ring electrode 21 - RV ring electrode 30
- Voltage measurement: RA tip electrode 20 - RV tip electrode 28

A second impedance vector in this preferred embodiment is:
- Current injection: RV ring electrode 30 - RV tip electrode 28
- Voltage measurement: RV ring electrode 30 - RV tip electrode 28

By measuring the cardiac impedance using these two vectors, the cardiac impedance signal will contain information both about local (the second vector) and global (the first vector) cardiac contraction characteristics. Further, according to the present invention, the cardiac impedance is measured at different activity levels, for example, at rest and at a physical effort.

A cardiac status indicator module 48 (see Fig. 3), preferably arranged in the cardiac stimulator 10, may use the hemodynamical information (e.g. the measured impedance waveforms) to extract hemodynamical parameters including maximum slope of a impedance waveform, area of a waveform during a heart cycle, and/or peak-to-peak value for a waveform. Based on the extracted hemodynamical parameters, a cardiac status indicator can be created using multivariate model. The cardiac status indicator indicates a response of the heart 12 of the patient 5 to an increased physical activity, for example, the response may be a primarily heart rate response or as a primarily a stroke volume response, or a combination between these two extremes.

Referring to Fig. 1 - 3, as indicated above, the cardiac stimulator 10 is configured to communicate with extracorporeal equipment, such as a programmer unit or workstation 6. The programmer unit 6 may comprise a control unit (not shown), a memory unit (not shown), communication unit (e.g. a telemetry unit) (not shown), and a display unit (not shown).

Furthermore, the external equipment 7 capable of measuring hemodynamical signals of the heart 12 of the patient 5 is configured to communicate with the programmer unit 6 and, optionally, with the cardiac stimulator 10, e.g. wirelessly including near-filed or far-field telemetry. A physician is enabled to monitor and observe, for example, cardiac response to increased workload via the external equipment 7. The non-implantable equipment 7 is not intended for chronic implantation and may include at least one or a combination of the equipment presented below. The list of example equipment given below is non-exhaustive. Further, examples of hemodynamical signals measured with respective equipment are also given together with examples of hemodynamical parameters that may be used to create the cardiac status indicator:
- non-invasive echocardiography/ultrasound equipment: This equipment may be based on ultrasound crystals generating pulses from a hand held probe that reflects tissue properties. By automatically moving the ultrasound beam, a 2D picture can be produced from which parameters can be derived. An examples of a parameter is aortic velocity time integral (correlates with stroke volume (SV))
- Non-invasive equipment for measurement using the fingers of the patient: This equipment may include a finger-cuff photoplethysmography technique. Using this technique, for example, stroke volume and LV dP/dtₘₐₓ can be obtained.
- Non-invasive magnetic resonance imaging (MRI): This technique uses a magnetic field to align the nuclear magnetization of hydrogen atoms in water in the body. Moving pictures are produced that can be quantified in same way as echocardiography images to quantify dyssynchrony or ejection-fraction or stroke volume.
- Swan-Ganz catheter: This technique measures cardiac output by monitoring temperature changes after bolus injection of a cold liquid. The injection is made into a proximal port of a catheter containing a thermistor mounted at the tip, which normally is placed in the pulmonary artery. The thermistor measures the sequential changes in temperature over time. The cardiac output is inversely related to the area under a plotted curve over the thermodilution. This is a standard method for monitoring cardiac output. Based on the measured cardiac output, stroke volume can be determined by dividing the cardiac output with heart rate.
- Catheter based equipment for measuring pulmonary capillary wedge pressure (left atrial pressure surrogate): Often a balloon-tipped, multi-lumen catheter (Swan-Ganz catheter) is inserted into a peripheral vein, advanced into right atrium, right ventricle, pulmonary artery and into a branch of the pulmonary artery. Behind the tip of the catheter, a small balloon is arrange, which may be inflated with air. The catheter has a first port at the distal tip and a second part placed more proximally relative to the balloon. The ports are connected to pressure transducers. When placed in a branch of the pulmonary artery, the distal port measures pulmonary artery pressure and the proximal port measures right atrial pressure. The balloon is then inflated, which occludes the branch of the pulmonary artery. When this occurs, the pressure in the distal port rapidly falls, and after a period of time, reaches a stable lower level that is similar to the left atrial pressure. Thereafter, the balloon is deflated. This catheter can also be used to measure cardiac output.
- Millar catheter or Radi pressure wire for measuring left ventricular pressure: For example, the left ventricular pressure can be measured by catheterization of the left ventricle e.g. through the femoral artery and a retrograde aortic valve. Using the left ventricle pressure signals, LV dP/dtₘₐₓ can be achieved..
- Equipment for impedance cardiography: For example, stroke volume (SV) or cardiac output (CO) can be obtained.

Turning now to Fig. 3, an exemplary, simplified block diagram depicting various components of the cardiac stimulator according to embodiments of the present invention is shown. The cardiac stimulator 10 is capable of delivering cardiac therapy and is configured to integrate both monitoring and therapy features, as will be described below. The cardiac stimulator 10 collects and processes data about the heart 12 from one or more sensors including hemodynamical sensors (e.g. an impedance measuring module) arranged in the cardiac stimulator and/or at least one hemodynamical sensor 15 arranged externally the cardiac stimulator 10. Further, the cardiac stimulator 10 collects and processes data about the heart 12 from electrode pairs for sensing cardiac electrogram (EGM) signals. While a particular multi-chamber device is shown, it is to be appreciated and understood that this is done for illustration purposes only. Thus, the techniques and methods described below can be implemented in connection with any suitable configured or configurable stimulation device. Accordingly, one of skill in the art could readily duplicate, eliminate, or disable the appropriate circuitry in any desired combination to provide a device capable of treating the appropriate chamber with pacing stimulation including cardiac resynchronisation therapy.

The cardiac stimulator 10 has a housing 40, often referred to as the "can" or "case electrode". The housing 40 may function as a return electrode in "unipolar" modes. Further, the housing 40 includes connector (not shown) having a plurality of terminals (not shown) for connection with electrodes and/or sensors.

The cardiac stimulator 10 includes a programmable microcontroller or control module 41 that inter alia controls the various modes of stimulation therapy. As well known within the art, the microcontroller 41 typically includes a microprocessor, or equivalent control circuitry, designed specifically for controlling the delivery of stimulation therapy and may further include RAM or ROM memory, logic and timing circuitry, state machine circuitry, and I/O circuitry. Typically, the microcontroller 41 includes the ability to process or monitor input signals (data or information) as controlled by a program stored in a designated block of memory. The type of microcontroller is not critical to the described implementations. Rather, any suitable microcontroller 41 may be used that carries out the functions described herein. The use of micro-processor based control circuits for performing timing and data analysis is well known in the art.

Furthermore, the cardiac stimulator 10 includes pacing module 42 adapted to provide pacing signals for delivery to the patient. The pacing module 42 comprises an atrial pulse generator 43 and a ventricular pulse generator 44 that generate pacing stimulation pulses for delivery by the right atrial lead 14, the coronary sinus lead 16, and/or the right ventricular lead 18 via an electrode configuration switch 45. It is understood that in order to provide stimulation therapy in each of the four chambers, the atrial and ventricular pulse generators 43 and 44, may include dedicated, independent pulse generators, multiplexed pulse generators, or shared pulse generators. The pulse generators 43 and 44 are controlled by the microcontroller 41 via appropriate control signals to trigger or inhibit stimulation pulses.

The microcontroller 41 further includes timing control circuitry 46 to control timing of the stimulation pulses (e.g. pacing rate, AV delay, VV delay, etc.) as well as to keep track of timing of refractory periods blanking intervals, etc. which is well known in the art. In addition, the microcontroller 41 may include components such as e.g. an arrhythmia detector (not shown) and/or a morphology detector (not shown). The aforementioned components may be implemented as part of the microcontroller 41, or as software/firmware instructions programmed into the device and executed on the microcontroller 41 during certain modes of operation.

Sensing circuits (not shown) comprising atrial sensing circuits and ventricular sensing circuits may also be coupled to the right atrial lead 14, the coronary sinus lead 16, and the right ventricular lead 18 through the switch 45 for detecting the presence of cardiac activity in each of the four chambers of the heart. Accordingly, the atrial sensing circuits and ventricular sensing circuits may include dedicated sense amplifiers, multiplexed amplifiers, or shared amplifiers.

The output from the atrial sensing circuits and ventricular sensing circuits are connected to the microcontroller 41, which, in turn, is able to trigger or inhibit the atrial sensing circuits and ventricular sensing circuits in a demand fashion in response to the absence or presence of cardiac activity in the appropriate chamber of the heart.

Furthermore, the microcontroller 41 is coupled to a memory 49 by a suitable data/address bus (not shown), wherein the programmable operating parameters used by the microcontroller 41 are stored and modified, as required, in order to customize the operation of the cardiac stimulator to the needs of a particular patient. Such operating parameters define, for example, pacing pulse amplitude, pulse duration, etc. Advantageously, the operating parameters may be non-invasively programmed into the memory 49 through a communication module 52 including, for example, a telemetry circuit for telemetric communication via communication link 53 with external devices 6, 7. The telemetry circuit 52 advantageously allows intracardiac electrograms and status information relating to the operation of the device 10 to be sent to the external devices 6, 7 through an established communication link 53. Further, the communication module may alternatively or as a complement to the telemetry circuit include circuit for RF communication.

The cardiac stimulator 10 may further include a sensor 56, commonly referred to as a "rate-responsive" sensor because it is typically used to adjust pacing stimulation rate according to the exercise state of the patient. While shown as being included within the stimulator 10, it is to be understood that the sensor 56 also may be external to the stimulator, yet still be implanted within or carried by the patient. Examples of such sensors include sensors that, for example, sense respiration rate, or activity sensors for sensing activity variance.

Moreover, the cardiac stimulator 10 additionally includes a battery 58 that provides operating power to all of the circuits shown in Fig. 2. Preferably, the stimulator 10 employs lithium or similar battery technology.

As mentioned above, the cardiac stimulator comprises a hemodynamical measuring module 47, in this embodiment an impedance measuring module, including a current injection circuit (not shown) for injecting current via the electrode configuration switch 45 and electrodes arranged in the medical leads (e.g. via RA ring electrode 21 and RV ring electrode 30 or via RV ring electrode 30 and RV tip electrode 28). Further, the impedance measuring module according to this embodiment further comprises a voltage measuring circuit (not shown) for measuring a voltage via the electrode configuration switch 45 and electrodes arranged in the medical leads (e.g. over RA tip electrode 20 and RV tip electrode 28 or via RV ring electrode 30 and RV tip electrode 28). The measured cardiac impedance signals can be used to determine a cardiac response of the heart of the patient to changed activity by comparing mechanical work of the heart 12 (reflected by the impedance waveforms) at each of at least two different levels of physical activity, wherein at least one level of physical activity corresponds to an increased activity level compared to an activity level at rest.

Further, the cardiac stimulator 10 comprises a cardiac status indicator module 48 creating a current cardiac status indicator for the patient 5 using measured hemodynamical signals reflecting mechanical work of the heart 12. The cardiac status indicator indicates a response of the patient to an increased physical activity, for example, as a primarily heart rate response or as a primarily a stroke volume response, or as combination between these two extremes. As mention above, the hemodynamical information at the different activity levels (e.g. the measured impedance waveforms) can be used to extract hemodynamical parameters including maximum slope of a impedance waveform, area of a waveform during a heart cycle, and/or peak-to-peak value for a waveform. Based on the extracted hemodynamical parameters, the cardiac status indicator can then be created using a multivariate model, and in a preferred embodiment the extracted parameters are linearly combined.

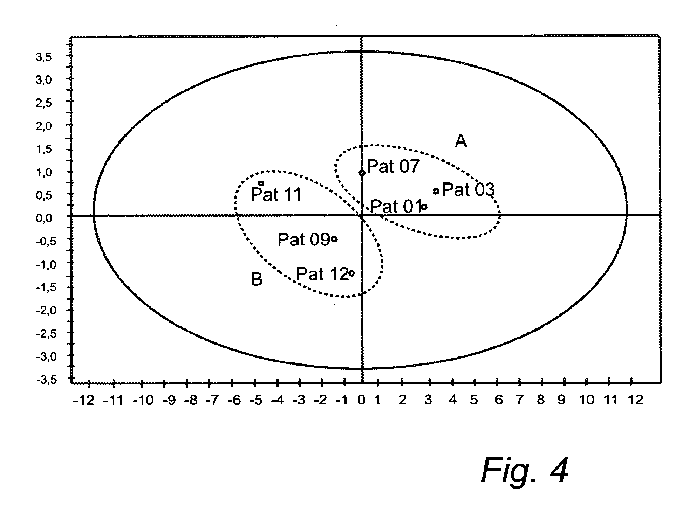

In Fig. 4, a multivariate plot using a multivariate model is shown. It is clearly illustrated how the two response groups are separated. The left ellipse includes patients having a primarily stroke volume response to increased activity and the right ellipse includes patients having a primarily heart rate response to the increased activity. Thus, the heart functions in different ways depending on which group the patient belongs to. On the horizontal axis, the value calculated by the multivariate model feed with hemodynamical data recorded at a first activity level (e.g. rest) is indicated, and on the vertical axis, the value calculated by the multivariate model feed with hemodynamical data recorded at a second activity level (e.g. at moderate physical exercise) is indicated. As can be seen, three patients, Pat 01, 03 and 07, have cardiac status indicators indicating a primarily heart rate response to the increased activity and patients, Pat 09, 11 and 12, have cardiac status indicators indicating a primarily stroke volume response to increased activity. Over time, a specific patient can move within a group and even from one group to the other, which reflects the cardiac status development of the patient. Of course, many patients have a combination of the two extreme responses but where one of the responses, i.e. primarily heart rate response or primarily stroke volume response, is more pronounced.

Moreover, the cardiac stimulator 10 comprises a pacing parameter determining module 62 configured to determine a pacing parameter setting based on a current cardiac status indicator, wherein the adapted pacing parameters include a first pacing setting if the current cardiac status indicator indicates a primarily heart rate response or a second pacing setting if the current cardiac status indicator indicates a primarily stroke volume response. For example, the first pacing setting may include a lower maximum track rate if a current cardiac status indicator indicates a primarily heart rate response and a second pacing setting may include a maintained slope or an increased slope of a rate response setting if a current cardiac status indicator indicates a primarily stroke volume response. Thus, an exercise mode can be created, i.e. specialized pacing modes for increased physical activity or exercise.

Patients having a primarily heart rate response to increased workload would benefit from a lower maximum track rate as they may have a tendency to rush the heart and worsen (often) the ischemic condition. Further, an exercise alert can be implemented that indicates for the patient that the heart rate has increased and thus advices the patient to slow down. A further option may be to apply optimized BiV pacing for ischemia as long as the patient is classified in patient group having primarily heart rate response.

Patients having a primarily stroke volume response on the other hand may be paced such that increased is promoted due to activity by increasing the slope of the rate response setting, such that a higher rate is employed during physical exertion. Furthermore, patients in this group often suffers from dilated cardiomyopathy and are likely to be very sensitive to myocardial wall stress, a condition caused by overload of blood volume or pressure. By using blood volume controlled pacing as described in US 5,417,715 and US 6,078,835 by the same applicant this problem can be handled.

The pacing parameter determining module 62 is further configured to instruct the timing circuitry 46 and pacing module 42 to adapt the pacing parameters depending on the current cardiac status indicator as suggested above.

Furthermore, an alarm device 64 is configured to, at receipt of an indication that an increased heart activity parameter and/or a changed activity of the patient has been detected, providing the patient with an alert informing the patient that the activity level is sufficiently high to cause the primarily heart rate response. The alarm device 64 may, for example, be a vibration unit adapted to vibrate to inform the patient of the increased activity level connected to the cardiac stimulator 10. The alarm device 64 is configured to, at receipt of an indication that a heart rate above a predetermined level has been detected, provide a patient having a cardiac status indicator indicating primarily a heart rate response with an alert suggesting the patient to reduce a level of activity at detection of the heart rate above the predetermined level.

With reference now to Fig. 5, one method adapting a pacing therapy of a cardiac stimulator implanted in a patient will be described.

First, at step S100, an adaptation session for changing or adapting pacing parameters of the cardiac stimulator is triggered or initiated by detection of at least one event related to the activity parameter, by receipt of an instruction to adapt the pacing parameters, or at regular intervals of time (e.g. once a week, or every 10-day period). For example, the adaptation session can be initiated on detection of an activity level being above a predetermined limit.

At step S110, a cardiac response of the heart of the patient to changed activity is determined by comparing mechanical work of the heart at each of at least two different levels of physical activity. To this end, at least one activity parameter reflecting physical activity of the patient is detected. The activity level of the patient may be continuously monitored and detected or at initiation of an adaptation session. In the latter case, an activity level detection step can be included between steps S100 and S110. If the activity of the patient is monitored and detected on continuous basis, an adaptation session may be initiated at, for example, at detection of an activity level above a predetermined level. In embodiments of the present invention, a cardiac response is determined using at least one hemodynamical parameter or signal recorded at each of at least two different levels of physical activity. As discussed above, cardiac impedance signals can be used in the determination of the cardiac response, which can be performed automatically within the cardiac stimulator 10. In an alternative embodiment, the cardiac response is determined by means of observations of a cardiac response at each of at least two different levels of physical activity, which includes observing a heart rate response and/or stroke volume response to changed activity. This can be performed by a physician using external monitoring/recording/detecting equipment 7, as has been discussed above. Preferably, the measurements and/or observations are conducted at rest and at a physical activity corresponding to an increased activity level compared to the activity level at rest.

Thereafter, at step S120, a current cardiac status indicator for the patient 5 using the cardiac response is created, which cardiac status indicator indicates a response of the patient to an increased physical activity as a primarily heart rate response or as a primarily a stroke volume response.

At step S130, the pacing parameters the cardiac stimulator 10 is adapted depending on a current cardiac status indicator. The adapted pacing parameters include a first pacing setting if the current cardiac status indicator indicates a primarily heart rate response or a second pacing setting if the current cardiac status indicator indicates a primarily stroke volume response. In particular, the first pacing setting may include a lower maximum track rate if the cardiac status indicator indicates a primarily heart rate response and the second pacing setting may include a maintained slope or an increased slope of a rate response setting if the current cardiac status indicator indicates a primarily stroke volume response.

It is intended that the scope of the present invention should not be limited by the particular embodiments described above, but should be determined by a fair reading of the claims that follow.

## Claims

1. A system (1) for adapting a pacing therapy of a cardiac stimulator (10) implanted in a patient (5), comprising:
an activity detector (56) configured to detect at least one activity parameter reflecting physical activity of said patient;
a hemodynamical measuring module (47) configured to measure hemodynamical signals at each of at least two different levels of physical activity said signals reflecting a cardiac response of the heart (12) of said patient (5) to changed activity, wherein at least one level of physical activity corresponds to an increased activity level compared to an activity level at rest;
a cardiac status indicator module (48) configured to create a current cardiac status indicator for said patient (5) using said cardiac response, wherein said cardiac status indicator indicates a response of said patient (5) to an increased physical activity as a primarily heart rate response or as a primarily a stroke volume response; and
a pacing parameter determining module (62) adapted to:
determine a pacing parameter setting based on said current cardiac status indicator wherein said adapted pacing parameters include a first pacing setting if said current cardiac status indicator indicates a primarily heart rate response or a second pacing setting if said current cardiac status indicator indicates a primarily stroke volume response; and
instruct a timing circuitry (46) and/or a pacing module (42) of said cardiac stimulator (10) to adapt pacing parameters depending on said current cardiac status indicator, wherein said adapted pacing parameters include a first pacing setting if said current cardiac status indicator indicates a primarily heart rate response or a second pacing setting if said current cardiac status indicator indicates a primarily stroke volume response.

2. The system according to claim 1, wherein said pacing parameter determining module (62) is configured to initiate an adaptation of therapy at receipt of an instruction to adapt the pacing parameters, or at regular intervals of time.

3. The system according to claim 1 or 2, wherein said pacing parameter determining module(62) is configured to initiate an adaptation of therapy at receipt of an indication that at least one event related to said activity parameter has occurred predetermined to trigger an adaptation.

4. The system according to claim 1-3, wherein said pacing parameter determining module (62) is configured to:
adapt pacing parameters of said cardiac stimulator (10) of said patient (5) depending on said current cardiac status indicator, wherein said first pacing setting includes a lower maximum track rate if said current cardiac status indicator indicates a primarily heart rate response and said second pacing setting includes a maintained slope or an increased slope of a rate response setting if said current cardiac status indicator indicates a primarily stroke volume response.

5. The system according to claim 1-4, further comprising:
an alarm device (64) configured to, at receipt of an indication that an increased heart activity parameter and/or a changed activity of said patient (5) has been detected, providing the patient (5) with an alert informing the patient (5) that the activity level is sufficiently high to cause said primarily heart rate response.

6. The system according to claim 5, wherein said alarm device (64) is configured to:
at receipt of an indication that a heart rate above a predetermined level has been detected, provide a patient (5) having a cardiac status indicator indicating primarily a heart rate response with an alert suggesting said patient (5) to reduce a level of activity at detection of said heart rate above said predetermined level.

7. The system according to claim 1 - 6, wherein said cardiac status indicator module (48) is configured to:
extract hemodynamical parameters from said hemodynamical signals, wherein at least one hemodynamical parameter is extracted from at least one hemodynamical signal measured during a measurement session performed when said activity parameter indicates said higher activity level;
determine said cardiac status indicator based on said extracted hemodynamical parameters; and
store said cardiac status indicator as a current status indicator.

8. The system according to claim 7, wherein said hemodynamical measuring module (47) is configured to record at least two hemodynamical signals using different hemodynamical sensors (56) during each measurement session, and wherein said cardiac status indictor module (48) is configured to extract at least one hemodynamical parameter from each hemodynamical signal for each measurement session.

9. The system according to claim 7 or 8, wherein said hemodynamical measuring module (47) is configured to record at least two cardiac impedance signals in each measurement session, wherein at least one cardiac impedance signal is measured using a first impedance measurement vector and at least one cardiac impedance signal is measured using a second impedance measurement vector.

10. The system according to claim 1-9, wherein said cardiac status indicator is configured to associate said current cardiac indicator to a value on a scale ranging from a first end value corresponding to a cardiac indicator indicating a response of said patient (5) to an increased physical activity as a primarily heart rate response to a second end value corresponding to a cardiac indicator indicating a response of said patient (5) to an increased physical activity as a primarily a stroke volume response.

11. The system according to claim 10, wherein said pacing parameter determining module (62) is configured to:
adapt pacing parameters of a cardiac stimulator (10) of said patient (5) depending on said value on said scale of said current cardiac status indicator, wherein a maximum track rate is lower the closer a value of said cardiac status indicator is to said first end value, and
wherein a slope of a rate response setting is set to be steeper the closer a value of said cardiac status indicator is to said second end value.

## Patentansprüche

1. System (1) zur Anpassung einer Stimulationstherapie eines Herzschrittmachers (10), der in einen Patienten (5) implantiert ist, umfassend:
einen Aktivitätsdetektor (56), der dafür konfiguriert ist, mindestens einen Aktivitätsparameter zu erfassen, welcher die körperliche Aktivität des Patienten widerspiegelt;
ein hämodynamisches Messmodul (47), welches dafür konfiguriert ist, auf jeder von mindestens zwei unterschiedlichen Stufen körperlicher Aktivität hämodynamische Signale zu messen, wobei die Signale eine Herzreaktion des Herzens des Patienten (5) auf veränderte Aktivität widerspiegeln, wobei mindestens eine Stufe körperlicher Aktivität einer erhöhten Aktivitätsstufe im Vergleich zu einer Aktivitätsstufe im Ruhezustand entspricht;
ein Herzzustandsindikatormodul (48), welches dafür konfiguriert ist, unter Verwendung der Herzreaktion für den Patienten (5) einen gegenwärtigen Herzzustandsindikator zu erzeugen, wobei der Herzzustandsindikator eine Reaktion des Patienten (5) auf eine erhöhte körperliche Aktivität als eine primäre Herzfrequenzreaktion oder als eine primäre Schlagvolumenreaktion anzeigt; und
ein Stimulationsparameter-Bestimmungsmodul (62), welches für das Folgende geeignet ist:
Bestimmen einer Stimulationsparametereinstellung auf der Grundlage des gegenwärtigen Herzzustandsindikators, wobei die angepassten Stimulationsparameter eine erste Stimulationseinstellung, wenn der gegenwärtige Herzzustandsindikator eine primäre Herzfrequenzreaktion anzeigt, oder eine zweite Stimulationseinstellung umfassen, wenn der gegenwärtige Herzzustandsindikator eine primäre Schlagvolumenreaktion anzeigt; und
Anweisen eines Zeitplanungs-Schaltungssystems (46) und/oder eines Stimulationsmoduls (42) des Herzschrittmachers (10), Stimulationsparameter in Abhängigkeit von dem gegenwärtigen Herzzustandsindikator anzupassen, wobei die angepassten Stimulationsparameter eine erste Stimulationseinstellung, wenn der gegenwärtige Herzzustandsindikator eine primäre Herzfrequenzreaktion anzeigt, oder eine zweite Stimulationseinstellung umfassen, wenn der gegenwärtige Herzzustandsindikator eine primäre Schlagvolumenreaktion anzeigt.

2. System nach Anspruch 1, wobei das Stimulationsparameter-Bestimmungsmodul (62) dafür konfiguriert ist, bei Empfang einer Anweisung zum Anpassen der Stimulationsparameter oder in regelmäßigen Zeitintervallen eine Anpassung der Therapie zu initiieren.

3. System nach Anspruch 1 oder 2, wobei das Stimulationsparameter-Bestimmungsmodul (62) dafür konfiguriert ist, eine Anpassung der Therapie bei Empfang einer Anzeige zu initiieren, dass mindestens ein auf den Aktivitätsparameter bezogenes Ereignis aufgetreten ist, welches dafür vorbestimmt ist, eine Anpassung auszulösen.

4. System nach Anspruch 1 bis 3, wobei das Stimulationsparameter-Bestimmungsmodul (62) für das Folgende konfiguriert ist:
Anpassen von Stimulationsparametern des Herzschrittmachers (10) des Patienten (5) in Abhängigkeit von dem gegenwärtigen Herzzustandsindikator, wobei die erste Stimulationseinstellung eine niedrigere maximale Verfolgungsfrequenz umfasst,
wenn der gegenwärtige Herzzustandsindikator eine primäre Herzfrequenzreaktion anzeigt, und die zweite Stimulationseinstellung eine beibehaltene Steilheit oder eine erhöhte Steilheit einer Frequenzreaktionseinstellung umfasst, wenn der gegenwärtige Herzzustandsindikator eine primäre Schlagvolumenreaktion anzeigt.

5. System nach Anspruch 1 bis 4, ferner umfassend:
eine Alarmvorrichtung (64), welche dafür konfiguriert ist, bei Empfang einer Anzeige, dass ein erhöhter Herzaktivitätsparameter und/oder eine veränderte Aktivität des Patienten (5) erfasst worden ist, dem Patienten (5) eine Warnmeldung zukommen zu lassen, welche den Patienten (5) informiert, dass die Aktivitätsstufe ausreichend hoch ist, um die primäre Herzfrequenzreaktion zu bewirken.

6. System nach Anspruch 5, wobei die Alarmvorrichtung (64) für das Folgende konfiguriert ist:
bei Empfang einer Anzeige, dass eine Herzfrequenz oberhalb einer vorgegebenen Stufe erfasst worden ist, Zukommenlassen einer Warnmeldung, mit welcher vorgeschlagen wird, dass der Patient (5) eine Aktivitätsstufe verringert, bei Erfassung der Herzfrequenz oberhalb der vorgegebenen Stufe an einen Patienten (5), der einen Herzzustandsindikator aufweist, der eine primäre Herzfrequenzreaktion anzeigt.

7. System Nach Anspruch 1 bis 6, wobei das Herzzustandsindikatormodul (48) für das Folgende konfiguriert ist:
Extrahieren hämodynamischer Parameter aus den hämodynamischen Signalen, wobei mindestens ein hämodynamischer Parameter aus mindestens einem hämodynamischen Signal extrahiert wird, welches während einer Messperiode gemessen wird, die durchgeführt wird, wenn der Aktivitätsparameter die höhere Aktivitätsstufe anzeigt;
Bestimmen des Herzzustandsindikators auf der Grundlage der extrahierten hämodynamischen Parameter und
Speichern des Herzzustandsindikators als gegenwärtigen Herzzustandsindikator.

8. System nach Anspruch 7, wobei das hämodynamische Messmodul (47) dafür konfiguriert ist, unter Verwendung verschiedener hämodynamischer Sensoren (56) während jeder Messperiode mindestens zwei hämodynamische Signale aufzuzeichnen und wobei das Herzzustandsindikatormodul (48) dafür konfiguriert ist, aus jedem hämodynamischen Signal für jede Messperiode mindestens einen hämodynamischen Parameter zu extrahieren.

9. System nach Anspruch 7 oder 8, wobei das hämodynamische Messmodul (47) dafür konfiguriert ist, in jeder Messperiode mindestens zwei Herzimpedanzsignale aufzuzeichnen, wobei mindestens ein Herzimpedanzsignal unter Verwendung eines ersten Herzimpedanz-Messvektors gemessen wird und mindestens ein Herzimpedanzsignal unter Verwendung eines zweiten Herzimpedanz-Messvektors gemessen wird.

10. System nach Anspruch 1 bis 9, wobei der Herzzustandsindikator dafür konfiguriert ist, den gegenwärtigen Herzindikator einem Wert auf einer Skala zuzuordnen, die von einem ersten Endwert, der einem Herzindikator entspricht, der eine Reaktion des Patienten (5) auf eine erhöhte körperliche Aktivität als eine primäre Herzfrequenzreaktion anzeigt, bis zu einem zweiten Endwert reicht, der einem Herzindikator entspricht, der eine Reaktion des Patienten (5) auf eine erhöhte körperliche Aktivität als eine primäre Schlagvolumenreaktion anzeigt.

11. System nach Anspruch 10, wobei das Stimulationsparameter-Bestimmungsmodul (62) für das Folgende konfiguriert ist:
Anpassen von Stimulationsparametern eines Herzschrittmachers (10) des Patienten (5) in Abhängigkeit von dem Wert des gegenwärtigen Herzzustandsindikators auf der Skala, wobei eine maximale Verfolgungsfrequenz umso niedriger ist, je näher ein Wert des Herzzustandsindikators an dem ersten Endwert liegt, und
wobei eine Steilheit einer Frequenzreaktionseinstellung so eingestellt ist, dass sie umso steiler ist, je näher ein Wert des Herzzustandsindikators an dem zweiten Endwert liegt.

## Revendications

1. Système (1) pour adapter une thérapie de stimulation d'un stimulateur cardiaque (10) implanté chez un patient (5), comprenant :
un détecteur d'activité (56) configuré pour détecter au moins un paramètre d'activité reflétant l'activité physique dudit patient ;
un module de mesure hémodynamique (47) configuré pour mesurer les signaux hémodynamiques à chacun d'au moins deux niveaux différents d'activité physique, lesdits signaux reflétant une réaction cardiaque du coeur (12) dudit patient (5) à un changement d'activité, dans lequel au moins un niveau d'activité physique correspond à un niveau d'activité accru en comparaison à un niveau d'activité au repos ;
un module d'indication de l'état cardiaque (48) configuré pour créer un indicateur d'état cardiaque actuel relatif audit patient (5) en utilisant ladite réaction cardiaque, dans lequel ledit indicateur d'état cardiaque indique une réaction dudit patient (5) à une activité physique accrue sous la forme principalement d'une réaction en termes de fréquence cardiaque ou sous la forme principalement d'une réaction en termes de débit systolique ; et
un module de détermination du paramètre de stimulation (62) adapté pour:
déterminer un réglage du paramètre de stimulation en fonction dudit indicateur d'état cardiaque actuel, lesdits paramètres de stimulation adaptés comprenant un premier réglage de stimulation si ledit indicateur d'état cardiaque actuel indique une réaction principalement en termes de fréquence cardiaque ou un second réglage de stimulation si ledit indicateur d'état cardiaque actuel indique une réaction principalement en termes de débit systolique ; et
informer un circuit de temporisation (46) et/ou un module de stimulation (42) dudit stimulateur cardiaque (10) afin qu'ils adaptent les paramètres de stimulation selon ledit indicateur d'état cardiaque actuel, lesdits paramètres de stimulation adaptés comprenant un premier réglage de stimulation si ledit indicateur d'état cardiaque actuel indique une réaction principalement en termes de fréquence cardiaque ou un second réglage de stimulation si ledit indicateur d'état cardiaque actuel indique une réaction principalement en termes de débit systolique.

2. Système selon la revendication 1, dans lequel ledit module de détermination du paramètre de stimulation (62) est configuré pour lancer une adaptation de la thérapie à réception d'une instruction visant à adapter les paramètres de stimulation, ou à des intervalles de temps réguliers.

3. Système selon la revendication 1 ou la revendication 2, dans lequel ledit module de détermination du paramètre de stimulation (62) est configuré pour lancer une adaptation de la thérapie à réception d'une indication de la survenue d'au moins un événement lié audit paramètre d'activité, prédéterminé pour déclencher une adaptation.

4. Système selon les revendications 1 à 3, dans lequel ledit module de détermination du paramètre de stimulation (62) est configuré pour :
adapter les paramètres de stimulation dudit stimulateur cardiaque (10) dudit patient (5) selon ledit indicateur d'état cardiaque actuel, ledit premier réglage de stimulation comprenant une fréquence de suivi maximale inférieure si ledit indicateur d'état cardiaque actuel indique une réaction principalement en termes de fréquence cardiaque, et ledit second réglage de stimulation comprenant une pente constante ou une pente accrue d'un réglage de réaction en fréquence si ledit indicateur d'état cardiaque actuel indique une réaction principalement en termes de débit systolique.

5. Système selon les revendications 1 à 4, comprenant également :
un dispositif d'alarme (64) configuré pour, à réception d'une indication de la détection d'un paramètre d'activité cardiaque accrue et/ou d'un changement d'activité dudit patient (5), fournir au patient (5) une alerte informant le patient (5) que le niveau d'activité est suffisamment élevé pour causer ladite réaction principalement en termes de fréquence cardiaque.

6. Système selon la revendication 5, dans lequel ledit dispositif d'alarme (64) est configuré pour :
à réception d'une indication de la détection d'une fréquence cardiaque supérieure à un niveau prédéterminé, fournir à un patient (5), ayant un indicateur d'état cardiaque indiquant principalement une réaction en termes de fréquence cardiaque, une alerte suggérant audit patient (5) de réduire le niveau d'activité à la détection de ladite fréquence cardiaque supérieure audit niveau prédéterminé.

7. Système selon les revendications 1 à 6, dans lequel ledit module d'indication de l'état cardiaque (48) est configuré pour :
extraire des paramètres hémodynamiques desdits signaux hémodynamiques, au moins un paramètre hémodynamique étant extrait d'au moins un signal hémodynamique mesuré pendant une session de mesure réalisée quand ledit paramètre d'activité indique ledit niveau supérieur d'activité ;
déterminer ledit indicateur d'état cardiaque en fonction desdits paramètres hémodynamiques extraits ; et
enregistrer ledit indicateur d'état cardiaque en tant qu'un indicateur d'état actuel.

8. Système selon la revendication 7, dans lequel ledit module de mesure hémodynamique (47) est configuré pour enregistrer au moins deux signaux hémodynamiques en utilisant des capteurs hémodynamiques (56) différents pendant chaque session de mesure, et dans lequel ledit module indicateur de l'état cardiaque (48) est configuré pour extraire au moins un paramètre hémodynamique de chaque signal hémodynamique pour chaque session de mesure.

9. Système selon les revendications 7 ou 8, dans lequel ledit module de mesure hémodynamique (47) est configuré pour enregistrer au moins deux signaux d'impédance cardiaque dans chaque session de mesure, au moins un signal d'impédance cardiaque étant mesuré en utilisant un premier vecteur de mesure d'impédance et au moins un signal d'impédance cardiaque étant mesuré en utilisant un second vecteur de mesure d'impédance.

10. Système selon les revendications 1 à 9, dans lequel ledit indicateur d'état cardiaque est configuré pour associer ledit indicateur cardiaque actuel à une valeur sur une échelle allant d'une première valeur d'extrémité correspondant à un indicateur cardiaque indiquant une réaction dudit patient (5) à une activité physique accrue sous forme d'une réaction principalement en termes de fréquence cardiaque à une seconde valeur d'extrémité correspondant à un indicateur cardiaque indiquant une réaction dudit patient (5) à une activité physique accrue sous forme d'une réaction principalement en termes de débit systolique.

11. Système selon la revendication 10, dans lequel ledit module de détermination du paramètre de stimulation (62) est configuré pour :
adapter les paramètres de stimulation d'un stimulateur cardiaque (10) dudit patient (5) selon ladite valeur sur ladite échelle dudit indicateur d'état cardiaque actuel, une fréquence de suivi maximale étant inférieure quand une valeur dudit indicateur d'état cardiaque est plus proche de ladite première valeur d'extrémité, et
dans lequel une pente d'un réglage de la réaction en fréquence est réglée pour être plus pentue quand une valeur dudit indicateur d'état cardiaque est plus proche de ladite seconde valeur d'extrémité.
